# EUROPEAN PATENT SPECIFICATION

(11) **EP 3 868 040 B1**
(45) Date of publication and mention of the grant of the patent: **11.12.2024**
(21) Application number: 19787211.2
(22) Date of filing: 11.10.2019
(51) Int. Cl.: H04B 13/00, A61B 5/00, H04W 12/033, H04W 12/47

(54) **ON-BODY COMMUNICATION SYSTEM AND METHOD OF COMMISSIONING THE SAME**
AM KÖRPER GETRAGENES KOMMUNIKATIONSSYSTEM UND VERFAHREN ZU DESSEN INBETRIEBNAHME
SYSTÈME DE COMMUNICATION SUR LE CORPS ET PROCÉDÉ DE MISE EN SERVICE

(30) Priority: 16.10.2018 US 201862746281 P
(43) Date of publication of application: 25.08.2021
(73) Proprietor: Koninklijke Philips N.V., 5656 AG Eindhoven (NL)
(72) Inventor: VISWESWARA, Ashoka, Sathanur, 5656 AE Eindhoven (NL); MEFTAH, Mohammed, 5656 AE Eindhoven (NL); JOHNSON, Mark, Thomas, 5656 AE Eindhoven (NL)
(74) Representative: Philips Intellectual Property & Standards
(86) International application number: PCT/EP2019/077584
(87) International publication number: WO 2020/078842

(56) References cited:
- WO-A1-2011/056856
- WO-A1-2016/133813
- WO-A2-01/87014
- US-A1- 2011 019 824
- US-A1- 2012 146 795
- US-A1- 2013 110 008
- US-A1- 2017 319 096
- US-A1- 2018 000 344
- US-B2- 7 955 258
- MASOUD ROSTAMI ET AL: "Balancing security and utility in medical devices?", 20130529; 1077952576 - 1077952576, 29 May 2013 (2013-05-29), pages 1 - 6, XP058020080, ISBN: 978-1-4503-2071-9, DOI: 10.1145/2463209.2488750

## Description

### FIELD OF THE INVENTION

The embodiments disclosed herein relate to a secure on-body communication system employing acoustic signal generation. A method of commissioning an on-body communication system, and a body-mountable unit comprising an acoustic signal receiver for receiving a body-coupled acoustic signal from the body of a subject, are also provided.

### BACKGROUND OF THE INVENTION

Monitoring of body parameters of a subject can be performed using an on-body or in-body sensor system and/or device. Such a system and/or device may permit ongoing or long-term monitoring. The system typically comprises at least one sensor unit for measuring physiological parameter(s), for example an on-body patch or an in-body implant. The sensor unit may be removably attached to the patient's body to permit continuous monitoring. The monitored physiological parameters can include for example heart-rate, breathing rate, blood oxygen measurements, and/or other vital signs. The system typically also includes a further unit for communicating with the sensor unit(s). This may be for collecting data from the sensor unit(s) or for assisting localization and correct positioning of the unit.

Commissioning of on/in body wearable devices (herein referred to as body-mountable or wearable devices) is an important step in configuring the system. Such a step often has to be done before the system can be used. On/in body wearable devices often use a wireless communication medium to transfer collected data to a remote server, for example via an intermediate hub. For secure data transmission, the hub and the wearable device may exchange a common secret key or keys which will then be used for encrypting the data collected by the on/in-body sensor system and transmitted in a secure way to the hub and then to the remote server. The secret keys may also be used for other purposes such as pairing and authentication. It is important that the secret keys are transferred to the configuring device of the on/in-body system in a secure manner to ensure that the key values are not compromised.

There are different ways in which secret keys can be transferred to a node of the on/in-body system.

One possible method would be to pre-program the on/in-body units of the system with the secret key(s) at the point of manufacture, or at a later time at the hospital in a separate commissioning step. A list of keys for sharing with the various body-mountable units of the system may be stored in a hospital database so that a record is kept of the keys being used by the different units. For example, the paper: Cook, S.C., 2013. Dynamic Near Field Communication Pairing For Wireless Sensor Networks describes an example of this approach.

The papers: S.A. Camtepe and B. Yener. Combinatorial design of key distribution mechanisms for wireless sensor networks. Networking, IEEE/ACM Transactions on, 15(2):346{358}, April 2007; and Shih-I Huang, Shiuhpyng Shieh, and S.Y. Wu. Adaptive random key distribution schemes for wireless sensor networks. In D.T. Lee, S.P. Shieh, and J.D. Tygar, editors, Computer Security in the 21st Century, pages 91 {105. Springer US, 2005 describe different ways in which the secret keys can be generated and transferred.

A problem with the approach of pre-programming the secret keys during manufacture is that the manufacturer may be a third party whose security cannot be guaranteed. Hence, sharing the keys with the manufacturer may critically compromise the security of the key value(s). Furthermore, programming at hospital in a separate commissioning process leads to delay in patient work flow, which leads to inefficiency and user inconvenience. It is also susceptible to human errors.

A further possible approach is to transfer the secret key(s) to the body-mountable or wearable units of the system during commissioning. To limit the risk of interception of the signals exchanged with the body-mountable or wearable unit during this transfer, an out of band (OOB) communication channel may be used. An out-of-band channel may refer to a channel intendent of a main (in-band) communication channel. For example, this may be a short-range communication channel such as near field communication (NFC) or Bluetooth. An external device containing the keys is paired with the body-mountable or wearable sensor unit and the key may be then transferred. The paper Cook, S. C (see above) describes method for using an OOB channel to pair and transfer secret keys from a hub to a node device.

One problem with this approach, however, is that it requires hospital personal to first commission the body-mountable or wearable sensor unit in order to set up an NFC communication channel with the further unit of the system or an external unit. This requires specialist hardware. This process causes delay and also possible human error in the work flow. The commissioning step might alternatively be performed at home by the patient. However, this causes inconvenience to the patient, as it required storage and use of the specialist NFC hardware at home. Furthermore, supplying a specialist NFC commissioning device for each system (to be taken home by the patient) adds additional cost, especially if the device is only used once to transfer key value(s).

The paper: Zhong, L et al, 2007, June. OsteoConduct: Wireless body-area communication based on bone conduction. In Proceedings of the ICST 2nd international conference on Body area networks (p. 9). ICST discusses use of bone conduction as a medium for transferring data between on/in body wearable devices. It leverages the human musculoskeletal system to transmit data and interface users in a low-power, secure, non-intrusive fashion. This approach is based on employing a mechanical stimulus in the form of patterned acoustic vibration, generated either by human users or external stimulators. A simple acoustic receiver, such as an accelerometer or microphone is used to sense the transmitted signals.

Due to the mechanical nature of this communication medium, commissioning of the communication channel (as is required for e.g. NFC or Bluetooth) is not necessary. This hence helps to mitigate the problems highlighted by the OOB key transfer approach.

However, the technology described in the above paper for implementing acoustic conduction is very limited in terms of its practical applications. In particular, a limitation of acoustic conduction based communication (as opposed to electromagnetic based communication) is that reception of the signals is highly direction-sensitive. This means that strong signal reception using typical microphone or accelerometer based receiver units is difficult. In particular, if the accelerometer or the microphone is not properly oriented, the reception of the acoustic signal can be poor leading to improper transfer of keys and/or greater latency in key transfer. Application of this communication approach in practical systems to achieve reliable communication therefore remains a challenge.

US 2013/110008 discloses a method of communication between an implantable medical device (IMD) and an external device. In some examples, the external device transmits a signal that includes a communication key. One or more sensors of the IMD sense the signal that includes the communication key, and the IMD uses the communication key for coding the communication between the IMD and the external device. The one or more sensors that sensed the signal may also sense one or more patient characteristics.

### SUMMARY OF THE INVENTION

The invention is defined by the claims.

According to examples in accordance with the embodiments disclosed herein, there is provided a secure on-body communication system comprising:
a peripheral unit, comprising an acoustic signal generator and a coupled driver adapted to drive generation of data-encoded acoustic signals by the signal generator based on input data signals, the peripheral unit adapted for acoustically coupling the generated acoustic signals into the body of a subject;
a body-mountable unit comprising an acoustic signal receiver for receiving the body-coupled acoustic signals from the body, and a decoder for decoding the acoustic signals to generate output data signals, the signal receiver comprising an array of acoustic sensor elements having sensing parts exposed at a body-contact surface of the body mountable-unit;
wherein the driver is adapted to perform an initialization procedure comprising establishing communication with an external device via an off-body communication medium, acquiring from the remote device a secure key, and driving acoustic transmission of the secure key to the body-mountable device by means of the acoustic signal generator; characterized in that the array of acoustic sensor elements is a two dimensional array.

The embodiments disclosed herein relate to the use of body-conduction of acoustic signals as an approach for transferring the secret keys from a peripheral unit to a body mountable unit of a system. To avoid the problem of high direction-sensitivity in receiving the signals, an acoustic receiver of the body mountable unit is formed of an array of acoustic sensor elements disposed at a body-contact surface of the body-mountable unit. The use of such an array ensures that the acoustic signals can be reliably received regardless of the orientation of the body-mountable unit or of the exact relative body-positions of the body-mountable unit and the peripheral unit. This adds flexibility as it permits both the orientation and the body-contact positions of the two units to be reconfigured without changing settings of the system.

Acoustic coupling is particularly suitable for low data rate communication between implantable or wearable devices, especially as a secure and low power alternative to wireless body-area network technologies, such as Bluetooth. It avoids the need for a commissioning process between the peripheral unit and the body-mountable unit. The embodiments described herein provide for implementation of the approach with greatly increased reliability and flexibility.

The term acoustic in this disclosure may refer to all mechanical (e.g. vibrational) waves transmitted through a medium including but not limited to vibration, sound, ultrasound and infrasound.

The secure key is for example a security key for encoding data to be sent between the body-mountable unit and the peripheral unit. The secure key may for example comprise or be an encryption key. The secure key may be or comprise a (security) code, e.g. an encryption code.

The system includes a peripheral unit for transmitting acoustic signals and a body-mountable or wearable unit configured for receiving the signals. The peripheral unit may be a portable or mobile unit or the peripheral unit may be a body-mountable unit and/or included in a body-mountable unit, being configured for removable attachment to a part of the body. The peripheral unit may in either case be configured for making temporary contact with the skin of the body for acoustically coupling generated signals into the body.

For example, the peripheral unit may be a wearable device such as a limb-worn device (e.g., a wrist-mountable unit, a leg-mountable unit, an arm-mountable unit and/or the like), or may in some examples be a mobile computing device such as a smartphone or tablet.

The peripheral unit 12 may be for coupling the generated acoustic signals though body contact.

In further examples, the peripheral unit may for instance be in the form of a standing platform having an upper standing surface for supporting the user in a standing position, the peripheral unit being may be adapted to couple the generated acoustic signals into the body of the subject via said upper standing surface. The platform may comprise a weight scale for instance. The weight scale may be a smart weight scale. The weight scale may optionally be further be adapted to measure one or more further body parameters such as for instance body weight, body mass index (BMI), body composition, heart rate and Pulse Wave Velocity (PWV).

The body-mountable unit may in examples be an on-body unit for mounting to an external part of the body of a subject, or an in-body unit for insertion or implantation in the body of a subject. The body-mountable unit may be for mounting to an external surface of the body of the subject.

The acoustic sensor elements of the acoustic signal receiver may be for coupling the acoustic signals out of the body. The sensor parts of the acoustic sensor elements may be sensing surfaces for example. These sensing parts may be disposed at a body-contact surface of the signal receiver, for instance distributed across the surface. The sensor elements may be disposed, e.g. mounted, on the body contact surface, or may be partially embedded for instance in the body contact surface, with sensing parts exposed. The body contact surface may be a surface for making (e.g. temporary or removable, or alternatively persistent) contact with the body of the subject, for coupling acoustic signals from the body of the subject.

The secure key is received at the acoustic signal receiver as an acoustic input signal which is provided to the decoder for converting into an output data signal.

As noted, the body-mountable unit may comprise an array of acoustic sensor elements to provide multi-directional signal sensing. The acoustic signal receiver may hence be a multi-directional signal receiver and preferably may be an omnidirectional signal receiver.

To facilitate improved multi-directional sensitivity, the array of acoustic sensor elements is a two dimensional array.

In advantageous examples, the array of acoustic sensor elements may be an annular array. Preferably, this may be a closed annular array.

An annular array of sensor element may include an array in which the sensor elements forming it describe an annular shape and preferably a closed annular shape; the array may have the elements distributed in a (closed) annular shape.

The technical advantage of an annular shape is greater efficiency of parts: the angular scope of directional sensitivity is maximized, while minimizing the required number of sensor elements (by eliminating sensor elements in the 'central' region of the defined annulus). Each of the elements defining the annular array shape is able to receive signals from a slightly different potential source direction.

The acoustic sensor elements of the signal receiver unit may be microphone elements, and preferably may be MEMS microphone elements. The array of acoustic sensor elements may in this case be in the form of a single-chip MEMS microphone array. Use of MEMS microphone elements may facilitate both a compact receiver unit, minimizing bulk and form factor, and also may permit higher density of sensor elements, allowing for maximizing directional sensitivity.

Processing of the signal outputs of the multiple acoustic sensor elements in order to derive a single coherent data output may be achieved in different ways.

For example, each of the acoustic sensor elements may be adapted to generate an independent signal output on receipt of an acoustic signal, The decoder may be adapted to identify a strongest of the received signal outputs and to generate the output data signal based on decoding of said strongest signal output alone. The decoder for instance may directly receive the independent signal output generated by each of the acoustic sensor elements and identify the strongest of the received signals.

This approach has the effect of selecting the sensor element which has greatest directional alignment with the incoming signal. By deriving the output data signal based on this signal alone, integrity of the received signal and thus the secret key may be maximized.

The initialization procedure may comprise two steps: a first in which the secure key may be acquired by, for example, the peripheral unit from a remote device (e.g. external to the system) and a second in which this code may be then securely transferred to the body-mountable unit by means of the body-coupled acoustic signaling.

Optionally, the off-body communication medium used to facilitate communication between the peripheral unit and the external device may be a wireless communication medium. For example, it may make of use electromagnetic signal transmission. This may use any communication protocol, for instance Wi-Fi, ZigBee or Cellular network communication. In this way the initialization procedure may involve a hybrid communication approach, in which standard electromagnetic communication may be used to receive the secure key at the peripheral unit, but the more secure acoustic communication means may be used to transfer the key from the peripheral unit to the body-mountable unit.

The off-body communication medium may include a secure medium, such as a secure, i.e. encrypted, Wi-Fi medium. Hence, risk of compromising the security of the key may be reduced. However, for short range transmission, data transferred using the on-body acoustic medium is significantly more secure, and hence provides a technical advantage.

In some embodiments, the system may comprise an intermediate hub, and wherein the body-mountable device may be adapted to acquire the secure key from the external device via the intermediate hub. The intermediate hub may by way of example take the form of a mobile computing device such as a smartphone or tablet or may for instance take the form of a memory or data-store or computing device.

This may for example be via a wireless communication link, e.g. employing electromagnetic signal transmission, e.g. Wi-Fi or via the Internet. The external device may be a mobile computing device, e.g. a smartphone.

A further aspect provides a method of commissioning an on-body communication system,
the system comprises
   a peripheral unit, comprising an acoustic signal generator and a coupled driver adapted to drive generation of data-encoded acoustic signals by the signal generator based on input data signals,
the method comprising:
   establishing communication between the driver and an external device via an off-body communication medium;
   acquiring at the driver a secure key from the remote device; and
   driving acoustic transmission of the secure key to the body-mountable device by means of the acoustic signal generator;
   wherein the body-mountable unit comprises an acoustic signal receiver for receiving body-coupled acoustic signals from the body, and a decoder for decoding the body-coupled acoustic signals into output data signals, the signal receiver comprising a two-dimensional array of acoustic sensor elements.

The above method also assumes that the peripheral unit has been positioned so as to be acoustically communicable with the body-mountable unit.

According to one or more embodiments, the method may further include a step of positioning the peripheral unit in acoustic communication with the body-mountable unit. This step is performed at least in advance of driving acoustic transmission of the key.

The step in one set of embodiments may comprise placing the peripheral unit in contact with an external part (e.g. surface) of the user's body, at a position remote (i.e. spatially separated) from the body-mountable unit. This is for facilitating acoustic coupling of signals through the body, using the body as an acoustic communication medium. In further embodiments, this step may comprise placing the peripheral unit in contact with the body-mountable unit, for direct acoustic coupling between the two units.

In particular, according to one or more embodiments, the method may further comprise, in advance at least of driving acoustic transmission of the secure key, positioning the peripheral unit in contact with the body-mountable unit for facilitating direct acoustic coupling between the two units.

According to examples in accordance with an aspect of the embodiments disclosed herein, there is provided a body-mountable unit (e.g., an in/on-body device as described herein) that comprises:
an acoustic signal receiver for receiving at least one body-coupled acoustic signal from the body of a subject; and
a decoder for decoding the at least one acoustic signals to generate output data signals;
wherein a driver coupled to an acoustic signal generator of a peripheral unit is adapted to perform an initialization procedure comprising establishing communication with an external device via an off-body communication medium, acquiring from the external device a secure key, and driving acoustic transmission of the secure key to the wearable device by means of the acoustic signal generator; and
characterized in that the acoustic signal receiver comprises a two-dimensional array of acoustic sensor elements having sensor parts exposed at a body-contact surface of the wearable unit.

The peripheral unit may include the acoustic signal generator and the coupled driver may be adapted to drive generation of data-encoded acoustic signals by the acoustic signal generator based on input data signals.

The peripheral unit may be configured for acoustically coupling the generated acoustic signals into the body of the subject. And the array of acoustic sensor elements may be a two dimensional array, an annular array, and/or a closed annular array. The array of acoustic sensor elements may be in the form of a single-chip MEMS microphone array. The acoustic sensor elements may be adapted to generate an independent signal output on receipt of an acoustic signal, and the decoder described herein may be adapted to identify a strongest of the received signal outputs and to generate the output data signal based on decoding of said strongest signal output alone.

The peripheral unit may be configured for coupling the generated acoustic signals though body contact. And, in some embodiments, the peripheral unit may in the form of a standing platform, such as a weight scale for example, having an upper standing surface for supporting the user in a standing position. The peripheral unit may adapted to couple the generated acoustic signals into the body of the subject via said upper standing surface. The peripheral unite may be in the form of a body-mountable unit, such as a limb-worn mountable unit. A limb-worn mountable unit may include a wrist-mountable unit, a leg-mountable unit, an arm-mountable unit, and/or the like.

In some embodiments, the acoustic sensor elements of the signal receiver unit described herein may be microphone elements, and preferably MEMS microphone elements. And, in some embodiments, the off-body communication medium may be a wireless communication medium.

The wearable device may be configured to acquire the secure key from the external device via an intermediate hub. The wearable device may be an on-body unit for mounting to an external part of the body of the subject and/or an in-body unit for insertion or implantation in the body of a subject.

These and other aspects will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying schematic drawings, in which:
Fig. 1 shows an example system in accordance with one or more embodiments;
Fig. 2 shows a body-contact surface of an example body-mountable unit of an example system in accordance with one or more embodiments;
Fig. 3 illustrates in situ placement of components of an example system according to one or more embodiments;
Fig. 4 illustrates components of an example system comprising a peripheral unit in the form of a weighing scale;
Fig. 5 illustrates example network architecture for acquiring secure keys from an external deice according to one or more embodiments;
Fig. 6 depicts processing circuitry of an example MEMS microphone array as may be incorporated in systems according to one or more embodiments;
Fig. 7 is a block diagram of an example method for commissioning an on-body system according to one or more embodiments; and
Fig. 8 is a block diagram of a further example method for commissioning an on-body system according to one or more embodiments.

### DETAILED DESCRIPTION OF THE EMBODIMENTS

The embodiments will be described with reference to the Figures.

It should be understood that the detailed description and specific examples, while indicating exemplary embodiments of the apparatus, systems and methods, are intended for purposes of illustration only and are not intended to limit the scope. These and other features, aspects, and advantages of the apparatus, systems and methods will become better understood from the following description, appended claims, and accompanying drawings. It should be understood that the Figures are merely schematic and are not drawn to scale. It should also be understood that the same reference numerals are used throughout the Figures to indicate the same or similar parts.

The embodiments described herein provide an on-body communication system comprising a peripheral, e.g. portable, unit and body-mountable unit. The peripheral unit and body-mountable unit may be configured to communicate via body-coupled acoustic signals. This is utilized to facilitate secure transmission of a secure key from the peripheral unit to the body-mountable unit. The body-mountable unit may comprise an array of acoustic sensor elements disposed at a body-coupling surface of the body-mountable unit for receiving the transmitted acoustic signals, the array formation facilitating multi or omni directional sensing. The key may be transferred in an initialization procedure which may comprise establishing communication with an external device via an off-body communication medium, acquiring from the external device a secure key, and driving acoustic transmission of the secure key to the body-mountable device by means of an acoustic signal generator.

For secure communication between an in/on-body device (a body-mountable or wearable device) and a data collection hub such as a smart watch/smartphone or any other mobile device and finally to a remote server, the body-mountable device, the hub and the remote server may share a common set of secret keys as part of a system commissioning process. This key may be used for instance for data encryption, secure pairing, and/or device authentication. It is desirable to avoid transferring the secret keys from the hub to the body-mountable unit using traditional wireless communication since this is susceptible to third party attacks (hacking) which can compromise the key values.

Alternative solutions of pre-programming the keys in the body-mountable device during manufacture or configuring the keys manually in situ causes delays, inconvenience and potentially human errors in the procedure.

Embodiments described herein propose a method to securely transfer the secret keys to a body-mountable device from an external device such as a hospital database system via a peripheral unit of the system acting as a hub. The final stage of transfer from the peripheral unit to the body-mountable unit is performed using body-coupled acoustic signaling.

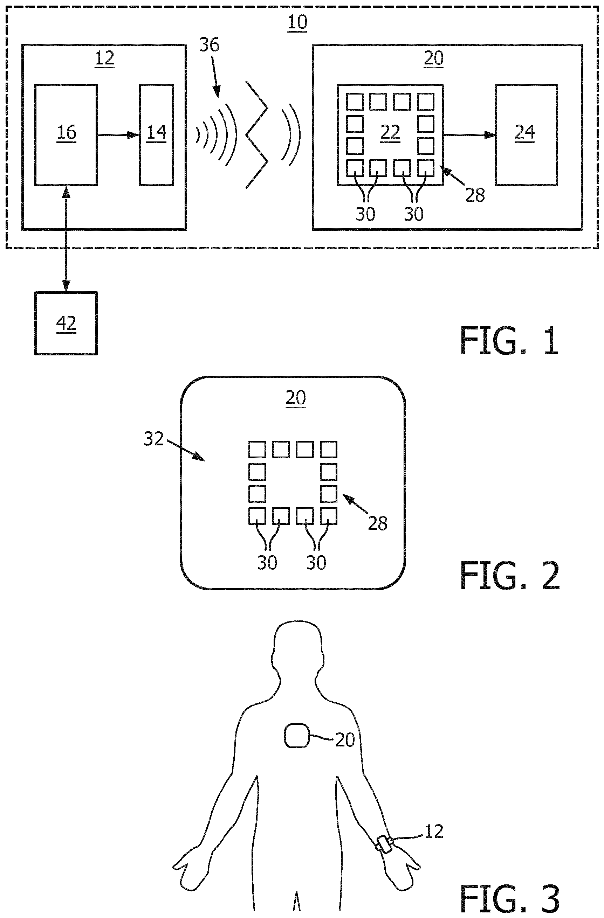

An example system 10 according to one or more embodiments is schematically depicted in Fig. 1.

The system 10 comprises two primary units: an on- or in-body unit (herein referred to as a body-mountable unit or a wearable device) 20 and a peripheral unit 12. The body mountable unit is configured for mounting to the body of a subject or for inserting or implanting in the body of the subject. It may be a wearable unit for example. The peripheral unit is configured for temporary placement in contact with the body of the subject, but may be for example a portable or mobile unit, configured to be removable from the body of the subject. It might be a unit carried by a member of hospital personal, or worn by them as a wearable device.

The peripheral unit 12 comprises an acoustic signal generator 14 and an operatively coupled driver 16 adapted to drive generation of data-encoded acoustic signals by the signal generator based on input data signals. The input data signals may be received from a local controller and/or local memory (not shown) for example and/or received through a communication link from an external source such as an external controller, server and/or data store.

The peripheral unit 12 is adapted for acoustically coupling the generated acoustic signals into the body of a subject. The peripheral unit is adapted for coupling the generated acoustic signals though body contact. The acoustic signal generator may comprise one or more acoustic transducers and/or acoustic actuators (e.g. loudspeakers) and may be adapted to generate encoded vibration waves in accordance with the drive signals received from the driver, and may be configured for applying these as an input stimulus to the subject's body (e.g. bone structure for example on the wrist and/or other body part and/or limb, or to the skin).

The body-mountable unit 20 comprises an acoustic signal receiver 22 for receiving the body-coupled acoustic signals from the body of the subject, and an operatively coupled decoder 24 for decoding the acoustic signals to generate output data signals. The acoustic signal receiver 22 comprises an array 28 of acoustic sensor elements 30 having sensing parts exposed at a body-contact surface 32 of the body mountable unit. Fig. 2 illustrates an example schematic representation of the components of the body-mountable unit. The actual spatial position of the components are exemplary and may vary.

Fig. 2 illustrates an example of an underside view of an example body mountable unit 20 (in this case illustrated as a patch) which shows the placement of the array 28 of acoustic sensor elements 30 having sensing surfaces exposed at a body-contact surface 32 of the unit. The sensing elements of the array may be disposed on the body-contact surface, or may be for example partially embedded or housed by the surface, with only acoustic sensing parts (e.g. sensing surfaces) exposed at the body-contact surface. The sensing surfaces may flush with the body-contact surface.

The system 10 is configured for implementing a commissioning or initialization procedure for the body-mountable unit 20 comprising transferring a patient or user specific secret key or keys from the peripheral unit 12, being placed in contact with the body, to the body mountable unit using acoustic body conduction techniques.

Accordingly, in use, the driver 16 of the peripheral unit 12 is adapted to perform an initialization or commissioning procedure comprising establishing communication with an external device 42 via an off-body (e.g. wireless) communication medium, acquiring from the external device a secure key, and driving acoustic transmission of the secure key to the body-mountable device 20 by means of the acoustic signal generator 14. Fig. 1 schematically illustrates and example propagation of generated data-encoded acoustic signals 36 from the peripheral unit 12 to the body-mountable unit.

The secure key(s) may for example include encryption keys for use by the body-mountable unit in encrypting data subsequently to be transmitted from the body-mountable unit to the peripheral unit. This data may for example be body-parameter related data.

When the signals are received at the signal receiver 22 of the body-mountable unit they may be passed to the decoder (for example a data-demodulation block) which extracts from the signals the encoded unique secret key or key values.

To perform the commissioning procedure, in use the body-mountable unit 20 may be mounted to the body, with the body-contact surface 32 held in contact against a tissue surface of the body. The body mountable unit may be an on-body unit, in which case it may be affixed, e.g. adhered to a skin surface of the body, or the unit may be an in-body (e.g. implantable) unit in which case, the unit may be held naturally against an internal surface or tissue section of the body by virtue of its implantation position.

The system commissioning procedure may be triggered for example by a user-input command to the peripheral unit 12 provided by the patient or a member of hospital personnel. In response to this input command, the peripheral unit may enable the acoustic actuator(s) and/or transducer(s) of the acoustic signal generator 14 and trigger the driver 16 to transmit the secret keys via vibration (e.g. audio) signals coupled to the underlying bone structure on the body and/or through the tissue. The secret keys may be transferred from the peripheral unit to the body-mountable unit 20 via the acoustic vibration (e.g. sound) waves transmitted through the bone or tissue conduction method through the body.

Although the system 10 is described herein as having only a single body-mountable unit 20, there may be provided more than one body mountable or wearable unit.

The body-mountable unit 20 may be configured for acquiring or sensing one or more body parameters for monitoring one or more body or physiological parameters.

As noted above, to ensure that the on- or in-body unit (body-mountable or wearable unit) 20 is able to receive the transmitted acoustic signals 36 irrespective of its orientation, an array 28 of acoustic sensor elements 30 may be used.

Preferably, a microphone sensor array may be used. In this case, each of the acoustic sensor elements 30 of the signal receiver unit may be a microphone element. Preferably, the array may be a MEMS (Microelectromechanical systems) based microphone sensor array, wherein each of the acoustic sensor elements is a MEMS microphone element.

The array of acoustic sensor elements may be preferably in the form of a single-chip MEMS microphone array. For example, all of the microphone elements may be formed on a single chip substrate or carrier. This may minimize form factor and permit a high sensor component density which may allow for maximized directional sensitivity to incoming acoustic signals.

The sensor elements 30 in the acoustic sensor array 28 may be positioned at the surface of the in/on-body device 20, facilitating capture of incoming acoustic signals irrespective of the device 20 orientation and/or relative location with respect to the peripheral unit. The signals captured by the array of sensor elements 30 may be first analyzed and the element with largest amplitude signal output may be selected and signals from this element then further processed (by the decoder 24) to decode the secret keys. This will be described in greater detail below.

To ensure maximal directional sensitivity, the acoustic sensor array 28 in the example of Fig. 1 is a two dimensional array. For example, the array may be a closed annular array (the sensor elements 30 of the array describe a closed annular shape). While the shape shown in Figs. 1 and 2 is a rectangular array, any other annular shape may be used, for example circular, oval, triangular, hexagonal.

By way of illustration, Fig. 3 schematically illustrates an example system 10 according to an embodiment, positioned in situ on a subject. The peripheral unit 12 may take the form of a wrist-mountable device, for example a smart-watch device. The body-mountable unit 20 may take the form of an externally body-mounted patch element 20 or any other in/on-body device as described herein. The configuration of Fig. 3 represents just one example structural implementation. In other examples, one or both of the body-mountable unit and the peripheral unit may take a different form.

For example, the peripheral unit 12 may comprise a different kind of body-mounted unit, for instance configured for attachment to a part of the body other than the wrist, such as the neck, chest, ear, leg, and/or abdomen. The peripheral unit may be an off-body unit, i.e. not configured for fixing to the body. For example, it may be a mobile computing device such as a smartphone, tablet, and/or other device. Where the peripheral unit is an off-body unit, it may be adapted for temporary application against a body-surface of the subject (e.g. the skin) in order to permit acoustic coupling of acoustic signals generated by the signal generator 14 into the body. The peripheral unit may have a dedicated body-contact surface for placing against the body to couple to signals into the body. For example, this may be aligned with transducers of the signal generator. Transducers of the signal generator may be disposed at the body-contact surface of the peripheral unit, or at least acoustically communicable with the surface.

The peripheral unit may comprise a standing platform having an upper standing surface for supporting the user in a standing position. The peripheral unit may be adapted to couple the generated acoustic signals into the body of the subject via an upper standing surface.

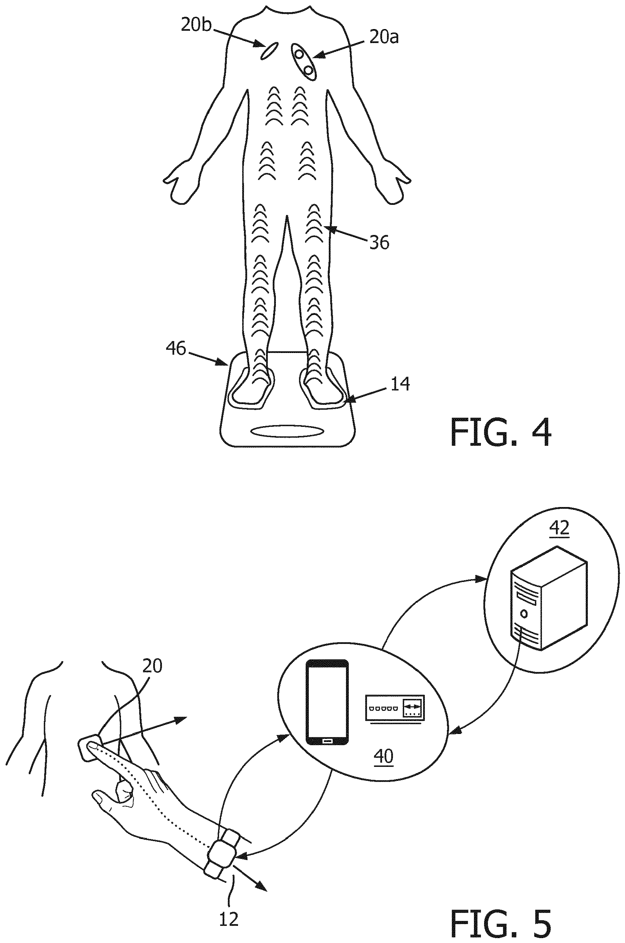

For example, in Fig. 4, the peripheral unit is in the form of a standing platform, such as a weight scale 46 having an acoustic signal generator 14 comprising vibrating transducers acoustically coupled with an upper surface of the scale. The upper surface may be arranged to receive a subject in a standing position on the scale. The transducers of the signal generator may be disposed in a respective arrangements extending across each of a pair of foot-receiving areas of the surface of the weight scale. In this way, the transducers may be located underneath the feet of the subject.

The transducers may be configured to generate vibrations 36 of sufficiently large amplitude to permit the signals to travel though the body towards (potentially multiple) wearable and/or insertable (body-mountable) units 20a, 20b. For example, vibrating mechanisms such as vibrating fitness plates (also known as power plates) may be used. These mechanisms may be configured for generating low frequency vibrations (around 50 Hz). However, the present embodiment is not limited to low frequencies of vibration, and higher frequencies may be used in examples.

The standing platform (e.g., weight scale) 46 may be a smart weight scale. A smart weight scale may be further configured for measuring one or more body parameters, such as weight, BMI, body composition, heart rate and Pulse Wave Velocity (PWV). Dedicated sensors and control circuitry may be integrated in the scale to facilitate this. The smart scale may be configured to connect wirelessly (e.g. via Bluetooth or Wi-Fi) to a user or subject's mobile device, for example smartphone or tablet. It may be configured to function as a communication hub for a wider network of in or on body health monitoring devices.

The body-mountable unit 20 may also take different forms. In the example of Fig. 3, the body-mountable unit may be in the form of a sensor patch unit for adhering to the skin of the patient. It may comprise an adhesive layer for instance covering at least a portion of a skin contact surface of the patch. The body-mountable unit may be any kind of on-body unit, e.g. a wearable unit such as a wrist-mountable unit or a unit configured for attachment to any other part of the body. The device may comprise an in-body unit for insertion or implantation in the body.

By way of illustration, Fig. 4 shows two example body-mountable units: an on-body type unit 20a for attaching to an external part of the body, and an in-body type unit 20b for inserting or implanting in the body.

As noted above, the system 10 initialization or commissioning procedure may comprise acquiring secure key(s) from an external device 42, such as from a database on a remote server. A schematic representation of an example data transfer path of the secure key(s) for the purposes of this acquisition is shown in Fig. 5.

In this example, the data path encompasses three different nodes of a data transfer network: the peripheral unit 12 (in this example, a wrist-mounted device), an intermediate hub 40 and the external device 42 (e.g. a remote server).

Fig. 5 shows an example network architecture for use with commissioning or initialization procedures described herein including the transfer of secret keys from a remote server 42 to a body-mountable unit 20 of the on-body system 10. In Fig. 5, the system may not yet being commissioned or initialized. The remote server 42 may generate a set of secret keys, each of which may include a relevant patient ID number. The set of keys may be then transferred to an intermediate hub 40, which may for example be in the form of a mobile computing device such as a smartphone and/or other device, or a (e.g. wirelessly) communicable computing hub, memory or data store, located proximal the patient. It may for example be within short-range wireless communication range of the peripheral unit, for example within range for communicating by Wi-Fi connection or Bluetooth or NFC connection for instance.

The intermediate hub 40 then may transfer the keys to the peripheral unit 12 which may act as a local hub in the network. By way of example, the peripheral unit 12 may be a wrist-mountable portable device or any other device as described herein. This device may be carried and/or worn by medical personnel for example and/or by the subject. This device may be incorporated into an in/on-body device. The wrist worn portable device may in other examples be adapted communicate directly with the remote server 42 (external device) in order to acquire the secure keys.

The communication medium between the remote server 42 and the intermediate hub 40 and between the intermediate hub and the peripheral unit 12 may by way of example be a standard wireless local area network (WLAN), wired local area network (LAN), a body area network (BAN), and/or another suitable communication platform. The peripheral unit 12, the intermediate hub 40 and the server may share a common set of keys such that their communication is fully secure.

Once the peripheral unit 12 has acquired the secure key(s), the key(s) may be transferred to the body-mountable unit 20 by first encoding the keys into acoustic waves such as sound waves for example, and then broadcasting or propagating these waves through the body by means of the acoustic signal generator 14 (e.g., and acoustic transducer and/or transducer assembly, a loudspeaker, and/or the like).

Optionally, the peripheral unit 12 may be touched by the subject and/or user to a point on the subject's body proximal to the body-location of the body-mountable unit 20 and/or touched onto the body-mountable unit itself.

The (sound) vibrations propagate through the bone structure and/or tissue of the subject to the body-mountable unit 40 and may be then captured by the array of acoustic sensor elements, e.g. motion sensors such as accelerometer or microphones, and decoded by a decoder. Once the secret keys are transferred to the body-mountable unit, the wearable device may perform a pairing procedure for pairing with the peripheral for the purpose of communicating for instance acquired body parameter information. This data may be exchanged in real time as it is acquired or it may be stored locally at the body-mountable device as it is acquired and subsequently transferred in one or more single operations at a later time.

As discussed above, to achieve reliable reception of acoustic signals irrespective of the orientation or location of the body-mountable unit on the subject's body, embodiments make use of an array of acoustic sensor elements disposed at a body-coupling surface of the body-mountable skin. The sensor array is preferably a MEMS based microphone sensor array which may be disposed across a surface area of the body mountable unit.

The paper: White, R.D et al, 2012. MEMS microphone array on a chip for turbulent boundary layer measurements. Proc. AIAA Aerosp. Sci. Meet, pp. 1-8 describes a suitable MEMS microphone array for use in embodiments. This paper describes the design, fabrication, characterization, and application of a suitable MEMS microphone array chip for the measurement of wall pressure spectra under turbulent boundary layers (TBL) in wind tunnel testing. This array may be used for the purpose of embodiments described herein. For example, MEMS sensor array-on-a-chip devices may allow high spatial resolution by co-fabricating multiple array elements side by side on the same die. This may allow for high directional sensitivity of the acoustic sensor element as the array can include a large number of sensor elements per unit angle around the array.

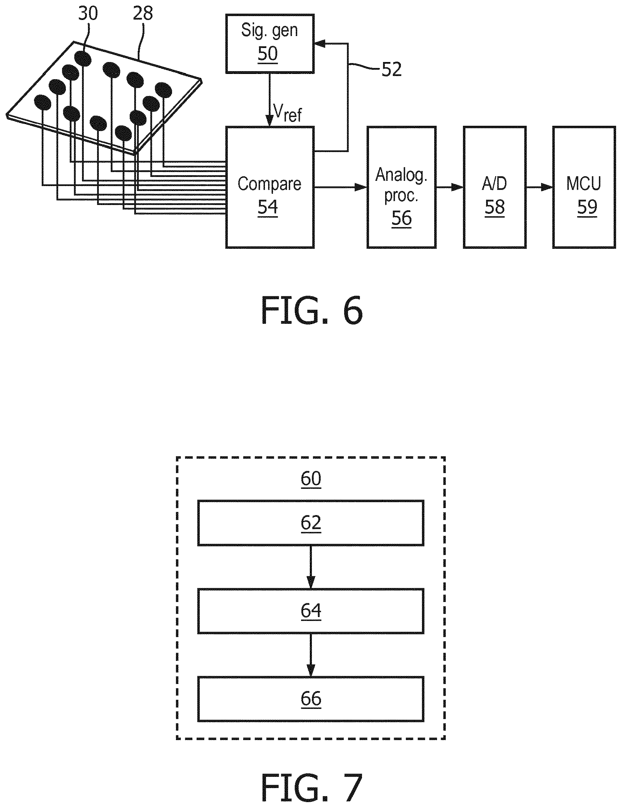

Fig. 6 schematically shows an example MEMS microphone sensor array 28. The array may be a single chip MEMS microphone array, with microphone elements 30 arranged in an annular array configuration. Fig. 6 also shows a block diagram of example signal processing circuitry for the acoustic sensor array 28.

Each of the acoustic sensor elements 30 of the array 28 may be adapted to generate an independent signal output on receipt of an acoustic signal. Each of the elements may be operatively coupled to a comparator/multiplexer block ("compare") 54 and may be configured to provide its respective signal output via said connection to the comparator/multiplexer block.

The comparator/multiplexor 54 may be adapted based on the set of received signal outputs to identify the sensor having the highest amplitude signal output voltage. A signal generator ("Sig. Gen") 50 may be adapted to supply to the comparator 54 a reference voltage V_{ref} of the correct voltage to cause the comparator circuit to de-select all of the received signal outputs apart from the identified one signal output having the highest amplitude.

The selected strongest sensor output signal may be passed through an analogue signal processing block (Analog. Proc") 56 to filter out-of-band signals to derive an amplified analogue signal. The filtered signal may be then passed through an acoustic to digital demodulator block ("A/D") 58 which may perform analogue to digital conversion and data demodulation. The resultant digital stream may be fed to a microcontroller unit ("MCU") 59 which may extract the encoded secure key(s) from the received input data stream.

In this example, the demodulator block 58 and the MCU 59 together may perform the decoding function of the decoder unit 24 of the body-mountable unit 20. By way of example, the decoder unit 24 may comprise the MCU and demodulator block. By way of example, the remaining signal processing circuitry (elements 50-56) may be comprised or housed or integrated as part of the acoustic signal receiver 22 of the body-mountable unit. Alternatively, all of the circuitry (elements 50-59) may be comprised by the decoder unit 24.

A method of commissioning an on-body communication system 10 may include the use of a peripheral unit 12, having an acoustic signal generator 14 and a coupled driver 16 adapted to drive generation of data-encoded acoustic signals 36 by the signal generator based on input data signals, and a body-mountable unit 20 having an acoustic signal receiver 22 for receiving the body-coupled acoustic signals from the body, and a decoder 24 for decoding the acoustic signals into output data signals, where the signal receiver may include an array 28 of acoustic sensor elements 30 having sensing parts exposed at a body-contact surface of the body mountable-unit.

A block diagram of an example method according to one or more embodiments is shown in Fig. 7.

The method 60 includes first establishing communication 62 between the driver 16 of the peripheral unit 12 and an external device 42 via an off-body communication medium. This may be a wireless communication medium such as, for example, wireless LAN, Wi-Fi, Bluetooth, BAN, and/or near field communication (NFC). The external device may be a remote server for example. The secure key may be stored in a database on the remote server. Following this, the method may include acquiring 64 at the driver 16 a secure key from the external device 42. The secure key may be understood in accordance with the explanations provided above with reference to the apparatus.

Once the secure key is acquired at the driver 16, the method includes driving 66 acoustic transmission of the secure key to the body-mountable device 20 of the system by means of the acoustic signal generator. This process may be performed in accordance with any of the procedures or means described above.

The above-outlined method 60 assumes that the body-mountable unit 20 has already been mounted on or in the body of the subject. According to more comprehensive embodiments, the method may further include a preliminary step of mounting the body-mountable unit on an external part of the body of a subject or inserting or implanting a body-mountable unit in the body of a subject. The above method 60 also assumes that the peripheral unit has been positioned so as to be acoustically communicable with the body-mountable unit. According to one or more embodiments, the method may further include a step of positioning the peripheral unit in acoustic communication with the body-mountable unit. This step is performed at least in advance of driving acoustic transmission of the key.

This positioning step in one set of embodiments may include placing the peripheral unit in contact with an external part (e.g. surface) of the user's body, at a position remote (i.e. spatially separated) from the body-mountable unit. This may facilitate acoustic coupling of signals through the body, using the body as an acoustic communication medium. Positioning may include placing the peripheral unit in contact with the body-mountable unit, for direct acoustic coupling between the two units.

In particular, according to one or more embodiments, the method may further comprise, in advance at least of driving acoustic transmission of the secure key, positioning the peripheral unit in contact with the body-mountable unit for facilitating direct acoustic coupling between the two units. This approach was depicted schematically for example in Fig. 5.

In this example, instead of acoustic signal conduction through the human body to the body-mountable unit 20, acoustic signals may be transferred directly from a (e.g. off-body) peripheral unit 12 to the body-mountable device by touching the peripheral unit to the on body wearable device or at a location on the body close to the in body wearable device.

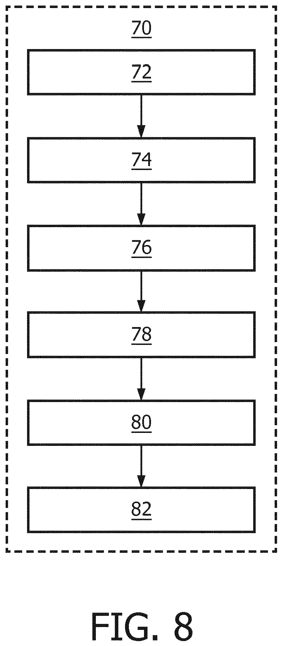

Fig. 8 shows an example block diagram of a method 70. In this method, the on-body commination system 10 may include or at least to be communicable with an intermediate hub device 40, for instance in the form of a smartphone or other computing device. The network architecture utilized in accordance with this method may for example be in accordance with the exemplary network architecture shown in the example of Fig. 5 and described above.

The method 70 may include in a first step 72 of establishing connection between the peripheral unit 12 and/or the intermediate hub 40. This may be via a wireless communication medium, for example short-range wireless communication such as via WLAN, BAN, Bluetooth, and/or NFC.

A second step 74 may include accessing at the peripheral unit 12, via the intermediate hub 40. Patient records data stored on a remote server 42 may be communicatively coupled with the intermediate hub. This step may comprise logging into the remote server using the peripheral unit and/or an auxiliary unit (e.g., a mobile computing device, a smartphone, a table, a laptop, and/or the like).

A third step 76 may include identifying the correct record for the patient in question (the subject to which the body-mountable unit is coupled) on the remote server and downloading the associated secure key(s) stored for the patient to the peripheral unit 12 via the intermediate hub 40. After logging in to the remote server the correct secure keys might be automatically identified and downloaded to the peripheral unit.

A next step 78 may include transferring the secure key from the peripheral unit 12 to the body-mountable unit 12 of the system 10 by, for example, placing the peripheral unit in contact with the body of the subject and initiating transfer of the keys via data-encoded acoustic signals coupled into the body and received at the body-mounted unit. The peripheral unit may be held in a position so that it is pressed against a bone of the patient, since acoustic coupling via the subject's bone structure leads to greater integrity in transferred messages and more reliable acoustic communication. The peripheral unit may be held in a position so that it is pressed against a tissue of a patient.

After transfer of the keys through the acoustic communication medium, the body-mountable device 20 may establish communication 80 with the peripheral unit 12 via an off-body (e.g. wireless) communication medium such as WLAN, NFC, BAN, or Bluetooth.

Using this off-body communication protocol, the body-mountable or wearable unit 20 may then transfer 82 collected data (for instance acquired body-parameter measurements) to the peripheral unit 12. The transferring data may be encrypted using the secure key. In this way, secure communication between the two units may be facilitated by means of the shared key.

The above disclosure has described a system and method for commissioning a secure on-body communication system, this commissioning procedure comprising transfer of a secure (e.g. encryption) key to a body-mounted device of the system using body-coupled acoustic signaling.

In summary, in accordance with one or more embodiments, a secure OOB (out of band) communication channel may be used. This channel may use a vibrating transducer and/or actuator capable of generating acoustic waves (e.g., a loudspeaker and/or the like). A body-mountable device may be embedded with an acoustic sensor array and associated electronics to receive and/or decode acoustic waves. The secure keys for the wearable device may be first transferred from an external device (e.g., a database such as a hospital database, a server, and/or the like) to a peripheral unit using secure traditional wireless communication technologies.

The secret keys may be converted into acoustic waves and broadcast through the body via a vibrating transducer. The transducer may be kept in contact with the body and the acoustic waves may propagate to the body-mounted unit via the body. Since the communication path may be restricted to the body structure of the patient/user in contact with the actuator device, the communication path provides a very secure means for implementing the key transfer. To enable reliable reception of the acoustic signals at the body-mountable unit 20, a MEMS microphone sensor array 28 may be used as the acoustic sensor.

Microphone sensor elements 30 of this array 28 may convert received input vibration to an electrical signal. For receiving the signals, the sensor elements of the array may be arranged with sensing parts exposed at a body-contact surface of the body-mountable unit so that the acoustic signals acoustically conduct from the body to the sensors (directly and/or indirectly). The electrical signal may be digitized, and passed through a data demodulation block to extract secure codes.

Embodiments described herein may provide a safe and efficient way of facilitating secure key exchange between the nodes of an on- or in-body system.

As discussed above, embodiments described herein provide for the transfer of secure keys via, for example, data encoded body-coupled acoustic signals. Detailed explanation and description of the encoding and decoding of data in acoustic signals, and of the mechanism for implementing body conduction of the encoded signals is provided in the paper: Zhong, L., et al, 2007, June. OsteoConduct: Wireless body-area communication based on bone conduction. In Proceedings of the ICST 2nd international conference on Body area networks (p. 9). ICST (Institute for Computer Sciences, Social-Informatics and Telecommunications Engineering).

Further detailed description of methods for data-encoding acoustic signals and conducting the signals through the human body are provided in US 8,867,994 B2, and in particular in the detailed description section of this publication.

Variations to the disclosed embodiments can be understood and effected by those skilled in the art in practicing the claimed embodiments, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A secure on-body communication system (10) comprising:
a peripheral unit (12), comprising an acoustic signal generator (14) and a coupled driver (16) adapted to drive generation of data-encoded acoustic signals by the signal generator based on input data signals, the peripheral unit adapted for acoustically coupling the generated acoustic signals into the body of a subject;
a body-mountable unit (20) comprising an acoustic signal receiver (22) for receiving the body-coupled acoustic signals from the body, and a decoder (24) for decoding the acoustic signals to generate output data signals, the signal receiver comprising an array (28) of acoustic sensor elements (30) having sensing parts exposed at a body-contact surface (32) of the body mountable unit;
wherein the driver (16) is adapted to perform an initialization procedure comprising establishing communication with an external device (42) via an off-body communication medium, acquiring from the external device a secure key, and driving acoustic transmission of the secure key to the body-mountable device by means of the acoustic signal generator;
**characterized in that** the array (28) of acoustic sensor elements (30) is a two dimensional array.

2. The system (10) as claimed in claim 1, wherein the array (28) of acoustic sensor elements (30) is an annular array, and preferably a closed annular array.

3. The system (10) as claimed in claim 1 or 2, wherein the peripheral unit (12) is for coupling the generated acoustic signals though body contact.

4. The system (10) as claimed in any of claims 1-3, wherein the acoustic sensor elements (30) of the signal receiver unit (22) are microphone elements, and preferably are MEMS microphone elements.

5. The system (10) as claimed in claim 4, wherein the array (28) of acoustic sensor elements (30) is in the form of a single-chip MEMS microphone array.

6. The system (10) as claimed in any of claims 1-5, wherein each of the acoustic sensor elements (30) is adapted to generate an independent signal output on receipt of an acoustic signal, and wherein the decoder (24) is adapted to identify a strongest of the received signal outputs and to generate the output data signal based on decoding of said strongest signal output alone.

7. The system (10) as claimed in any of claims 1-6, wherein the system comprises an intermediate hub (40), and wherein the body-mountable device (20) is adapted to acquire the secure key from the external device (42) via the intermediate hub.

8. The system (10) as claimed in any of claims 1-7, wherein the peripheral unit (12) is in the form of a standing platform (46) having an upper standing surface for supporting the user in a standing position, the peripheral unit (12) being adapted to couple the generated acoustic signals into the body of the subject via said upper standing surface, and
optionally wherein the platform (46) comprises a weight scale.

9. The system (10) as claimed in any of claims 1-8, wherein
the body-mountable unit 20 is an on-body unit for mounting to an external part of the body of a subject, for example a wrist-mountable unit; or
the body-mountable unit 20 is an in-body unit for insertion or implantation in the body of a subject.

10. A method (60, 70) of commissioning an on-body communication system (10), wherein the system comprises a peripheral unit (12), comprising an acoustic signal generator (14) and a coupled driver (16) adapted to drive generation of data-encoded acoustic signals (36) by the signal generator based on input data signals, the method comprising:
establishing (62) communication between the driver (16) and an external device via an off-body communication medium;
acquiring (64) at the driver (16) a secure key from the external device; and
driving (66) acoustic transmission of the secure key to a body-mountable unit (20) by means of the acoustic signal generator (14);
wherein the body-mountable unit (20) comprises an acoustic signal receiver (22) for receiving body-coupled acoustic signals from the body, and a decoder (24) for decoding the body-coupled acoustic signals into output data signals, the signal receiver comprising a two-dimensional array (28) of acoustic sensor elements (30).

11. The method (60, 70) as claimed in claim 10, wherein the two-dimensional array (28) of acoustic sensor elements (30) comprises sensing parts exposed at a body-contact surface of the body mountable-device.

12. The method (60, 70) as claimed in claim 10 or 11, further comprising, prior to driving acoustic transmission of the secure key, positioning the peripheral unit (12) in contact with the body-mountable unit (20) for facilitating direct acoustic coupling between the two units.

13. The body-mountable unit (20) comprising:
an acoustic signal receiver (22) for receiving at least one body-coupled acoustic signal from the body of a subject; and
a decoder (24) for decoding the at least one acoustic signals to generate output data signals;
wherein a driver (16) coupled to an acoustic signal generator (14) of a peripheral unit (12) is adapted to perform an initialization procedure comprising establishing communication with an external device (42) via an off-body communication medium, acquiring from the external device a secure key, and driving acoustic transmission of the secure key to the wearable device by means of the acoustic signal generator; and
**characterized in that** the acoustic signal receiver comprises a two-dimensional array (28) of acoustic sensor elements (30) having sensor parts exposed at a body-contact surface (32) of the body-mountable unit (20).

14. The body-mountable unit (20) as claimed in claim 13, wherein the peripheral unit (12) comprising the acoustic signal generator (14) and the coupled driver (16) is adapted to drive generation of data-encoded acoustic signals by the acoustic signal generator based on input data signals.

15. The body-mountable unit (20) as claimed in claim 13 or 14, wherein the body-mountable unit (20) is at least one of an on-body unit for mounting to an external part of the body of the subject or an in-body unit for insertion or implantation in the body of a subject.

## Patentansprüche

1. Sicheres am Körper getragenes Kommunikationssystem (10), umfassend:
eine Peripherieeinheit (12), die einen Akustiksignalgenerator (14) und einen gekoppelten Treiber (16) umfasst, der angepasst ist, das Erzeugen datencodierter Akustiksignale durch den Signalgenerator basierend auf Eingangsdatensignalen anzusteuern, wobei die Peripherieeinheit zum akustischen Koppeln der erzeugten Akustiksignale in den Körper einer Person angepasst ist;
eine am Körper anbringbare Einheit (20), die einen Akustiksignalempfänger (22) zum Empfangen der körpergekoppelten Akustiksignale vom Körper und einen Decoder (24) zum Decodieren der Akustiksignale umfasst, um Ausgabedatensignale zu erzeugen, wobei der Signalempfänger eine Anordnung (28) aus Akustiksensorelementen (30) umfasst, deren Sensorteile an einer Körperkontaktoberfläche (32) der am Körper anbringbaren Einheit freiliegen;
wobei der Treiber (16) angepasst ist, eine Initialisierungsprozedur durchzuführen, welche das Aufbauen von Kommunikation mit einer externen Vorrichtung (42) über ein körperfremdes Kommunikationsmedium, das Erfassen eines sicheren Schlüssels von der externen Vorrichtung und das Ansteuern einer Akustikübertragung des sicheren Schlüssels zur am Körper anbringbaren Vorrichtung anhand des Akustiksignalgenerators umfasst;
**dadurch gekennzeichnet, dass** die Anordnung (28) aus Akustiksensorelementen (30) eine zweidimensionale Anordnung ist.

2. System (10) nach Anspruch 1, wobei die Anordnung (28) aus Akustiksensorelementen (30) eine ringförmige Anordnung und vorzugsweise eine geschlossene ringförmige Anordnung ist.

3. System (10) nach Anspruch 1 oder 2, wobei die Peripherieeinheit (12) zum Koppeln der erzeugten Akustiksignale durch Körperkontakt dient.

4. System (10) nach einem der Ansprüche 1-3, wobei die Akustiksensorelemente (30) der Signalempfängereinheit (22) Mikrofonelemente, und vorzugsweise MEMS-Mikrofonelemente sind.

5. System (10) nach Anspruch 4, wobei die Anordnung (28) aus Akustiksensorelementen (30) in der Form einer Single-Chip-MEMS-Mikrofon-Anordnung ist.

6. System (10) nach einem der Ansprüche 1-5, wobei jedes der Akustiksensorelemente (30) angepasst ist, bei Empfang eines Akustiksignals eine unabhängige Signalausgabe zu erzeugen, und wobei der Decoder (24) angepasst ist, die stärkste der empfangenen Signalausgaben zu identifizieren und das Ausgabedatensignal allein basierend auf der Dekodierung der stärksten Signalausgabe zu erzeugen.

7. System (10) nach einem der Ansprüche 1-6, wobei das System einen Zwischen-Hub (40) umfasst und wobei die am Körper anbringbare Vorrichtung (20) angepasst ist, den sicheren Schlüssel über den Zwischen-Hub von der externen Vorrichtung (42) zu erfassen.

8. System (10) nach einem der Ansprüche 1-7, wobei die Peripherieeinheit (12) in der Form einer stehenden Plattform (46) ist, die eine obere stehende Oberfläche zum Stützen des Benutzers in einer stehenden Position aufweist, wobei die Peripherieeinheit (12) angepasst ist, die erzeugten Akustiksignale über die obere stehende Oberfläche in den Körper der Person zu koppeln, und
wobei die Plattform (46) optional eine Gewichtsskala umfasst.

9. System (10) nach einem der Ansprüche 1-8, wobei
die am Körper anbringbare Einheit 20 eine am Körper getragene Einheit zum Anbringen an einem äußeren Körperteil einer Person, beispielsweise eine am Handgelenk anbringbare Einheit ist; oder
die am Körper anbringbare Einheit 20 eine im Körper befindliche Einheit zum Einsetzen oder Implantieren in den Körper einer Person ist.

10. Verfahren (60, 70) zur Inbetriebnahme eines am Körper getragenen Kommunikationssystems (10),
wobei das System eine Peripherieeinheit (12) umfasst, die einen Akustiksignalgenerator (14) und einen gekoppelten Treiber (16) umfasst, der angepasst ist, das Erzeugen datencodierter Akustiksignale (36) durch den Signalgenerator basierend auf Eingangsdatensignalen anzusteuern, wobei das Verfahren umfasst:
Aufbauen (62) einer Kommunikation zwischen dem Treiber (16) und einer externen Vorrichtung über ein körperfremdes Kommunikationsmedium;
Erfassen (64) eines sicheren Schlüssels von der externen Vorrichtung beim Treiber (16) und Ansteuern (66) einer Akustikübertragung des sicheren Schlüssels an eine am Körper anbringbare Einheit (20) anhand des Akustiksignalgenerators (14);
wobei die am Körper anbringbare Einheit (20) einen Akustiksignalempfänger (22) zum Empfangen körpergekoppelter Akustiksignale vom Körper und einen Decoder (24) zum Decodieren der körpergekoppelten Akustiksignale in Ausgabedatensignale umfasst, wobei der Signalempfänger eine zweidimensionale Anordnung (28) aus Akustiksensorelementen (30) umfasst.

11. Verfahren (60, 70) nach Anspruch 10, wobei die zweidimensionale Anordnung (28) aus Akustiksensorelementen (30) Sensorteile umfasst, die an einer Körperkontaktoberfläche der am Körper anbringbaren Vorrichtung freiliegen.

12. Verfahren (60, 70) nach Anspruch 10 oder 11, das weiter vor dem Ansteuern der Akustikübertragung des sicheren Schlüssels das Positionieren der Peripherieeinheit (12) in Kontakt mit der am Körper anbringbaren Einheit (20) zum Erleichtern einer direkten akustischen Kopplung zwischen den beiden Einheiten umfasst.

13. Am Körper anbringbare Einheit (20), umfassend:
einen Akustiksignalempfänger (22) zum Empfangen von mindestens einem körpergekoppelten Akustiksignal vom Körper einer Person; und
einen Decoder (24) zum Decodieren des mindestens einen Akustiksignals, um Ausgabedatensignale zu erzeugen;
wobei ein Treiber (16), der mit einem Akustiksignalgenerator (14) einer Peripherieeinheit (12) gekoppelt ist, angepasst ist, eine Initialisierungsprozedur durchzuführen, welche das Aufbauen von Kommunikation mit einer externen Vorrichtung (42) über ein körperfremdes Kommunikationsmedium, das Erfassen eines sicheren Schlüssels von der externen Vorrichtung und das Ansteuern einer Akustikübertragung des sicheren Schlüssels zur tragbaren Vorrichtung anhand des Akustiksignalgenerators umfasst; und
**dadurch gekennzeichnet, dass** der Akustiksignalempfänger eine zweidimensionale Anordnung (28) aus Akustiksensorelementen (30) umfasst, deren Sensorteile an einer Körperkontaktoberfläche (32) der am Körper anbringbaren Einheit (20) freiliegen.

14. Am Körper anbringbare Einheit (20) nach Anspruch 13, wobei die Peripherieeinheit (12), die den Akustiksignalgenerator (14) und den gekoppelten Treiber (16) umfasst, angepasst ist, das Erzeugen datencodierter Akustiksignale durch den Akustiksignalgenerator basierend auf Eingangsdatensignalen anzusteuern.

15. Am Körper anbringbare Einheit (20) nach Anspruch 13 oder 14, wobei die am Körper anbringbare Einheit (20) mindestens eines ist von: einer am Körper tragbaren Einheit zum Anbringen an einem äußeren Körperteil der Person oder eine im Körper befindliche Einheit zum Einsetzen oder Implantieren in den Körper einer Person.

## Revendications

1. Système de communication sécurisée sur le corps (10) comprenant :
une unité périphérique (12), comprenant un générateur de signaux acoustiques (14) et un pilote couplé (16) adapté pour piloter une génération de signaux acoustiques codés en données par le générateur de signaux sur la base de signaux de données d'entrée, l'unité périphérique étant adaptée pour coupler acoustiquement les signaux acoustiques générés dans le corps d'un sujet ;
une unité montable sur le corps (20) comprenant un récepteur de signaux acoustiques (22) pour recevoir les signaux acoustiques couplés au corps en provenance du corps, et un décodeur (24) pour décoder les signaux acoustiques pour générer des signaux de données de sortie, le récepteur de signaux comprenant un réseau (28) d'éléments de capteur acoustique (30) présentant des parties de détection exposées au niveau d'une surface de contact avec le corps (32) de l'unité montable sur le corps ;
dans lequel le pilote (16) est adapté pour réaliser une procédure d'initialisation comprenant l'établissement d'une communication avec un dispositif externe (42) par le biais d'un support de communication hors corps, l'acquisition à partir du dispositif externe d'une clé sécurisée, et le pilotage d'une transmission acoustique de la clé sécurisée au dispositif montable sur le corps au moyen du générateur de signaux acoustiques ;
**caractérisé en ce que** le réseau (28) d'éléments de capteur acoustique (30) est un réseau bidimensionnel.

2. Système (10) selon la revendication 1, dans lequel le réseau (28) d'éléments de capteur acoustique (30) est un réseau annulaire et, de préférence, un réseau annulaire fermé.

3. Système (10) selon la revendication 1 ou 2, dans lequel l'unité périphérique (12) est destinée à coupler les signaux acoustiques générés par contact corporel.

4. Système (10) selon l'une quelconque des revendications 1-3, dans lequel les éléments de capteur acoustique (30) de l'unité de réception de signal (22) sont des éléments de microphone et, de préférence, sont des éléments de microphone MEMS.

5. Système (10) selon la revendication 4, dans lequel le réseau (28) d'éléments de capteur acoustique (30) se présente sous la forme d'un réseau de microphones MEMS monopuce.

6. Système (10) selon l'une quelconque des revendications 1-5, dans lequel chacun des éléments de capteur acoustique (30) est adapté pour générer une sortie de signal indépendante à la réception d'un signal acoustique et dans lequel le décodeur (24) est adapté pour identifier la sortie de signal la plus forte parmi celles reçues et pour générer le signal de données de sortie sur la base du décodage de ladite seule sortie de signal la plus forte.

7. Système (10) selon l'une quelconque des revendications 1-6, dans lequel le système comprend un concentrateur intermédiaire (40) et dans lequel le dispositif montable sur le corps (20) est adapté pour acquérir la clé sécurisée à partir du dispositif externe (42) par le biais du concentrateur intermédiaire.

8. Système (10) selon l'une quelconque des revendications 1-7, dans lequel l'unité périphérique (12) se présente sous la forme d'une plate-forme d'appui (46) présentant une surface d'appui supérieure pour supporter l'utilisateur en position debout, l'unité périphérique (12) étant adaptée pour coupler les signaux acoustiques générés dans le corps du sujet par le biais de ladite surface d'appui supérieure, et
facultativement dans lequel la plate-forme (46) comprend une balance.

9. Système (10) selon l'une quelconque des revendications 1-8, dans lequel
l'unité montable sur le corps (20) est une unité sur le corps destinée à être montée sur une partie externe du corps d'un sujet, par exemple une unité montable sur le poignet ; ou
l'unité montable sur le corps (20) est une unité intra-corporelle pour une insertion ou une implantation dans le corps d'un sujet.

10. Procédé (60, 70) de mise en service d'un système de communication sur le corps (10),
dans lequel le système comprend une unité périphérique (12), comprenant un générateur de signaux acoustiques (14) et un pilote couplé (16) adapté pour piloter une génération de signaux acoustiques codés en données (36) par le générateur de signaux sur la base de signaux de données d'entrée, le procédé comprenant :
l'établissement (62) d'une communication entre le pilote (16) et un dispositif externe par le biais d'un support de communication hors corps ;
l'acquisition (64), au niveau du pilote (16), d'une clé sécurisée à partir du dispositif externe ; et le pilotage (66) d'une transmission acoustique de la clé sécurisée à une unité montable sur le corps (20) au moyen du générateur de signaux acoustiques (14) ;
dans lequel l'unité montable sur le corps (20) comprend un récepteur de signal acoustique (22) pour recevoir des signaux acoustiques couplés au corps en provenance du corps, et un décodeur (24) pour décoder les signaux acoustiques couplés au corps en signaux de données de sortie, le récepteur de signal comprenant un réseau bidimensionnel (28) d'éléments de capteur acoustique (30).

11. Procédé (60, 70) selon la revendication 10, dans lequel le réseau bidimensionnel (28) d'éléments de capteur acoustique (30) comprend des parties de détection exposées au niveau d'une surface de contact avec le corps du dispositif montable sur le corps.

12. Procédé (60, 70) selon la revendication 10 ou 11, comprenant en outre, avant le pilotage d'une transmission acoustique de la clé sécurisée, le positionnement de l'unité périphérique (12) en contact avec l'unité montable sur le corps (20) pour faciliter un couplage acoustique direct entre les deux unités.

13. Unité montable sur le corps (20) comprenant :
un récepteur de signal acoustique (22) pour recevoir au moins un signal acoustique couplé au corps en provenance du corps d'un sujet ; et
un décodeur (24) pour décoder le au moins un signal acoustique pour générer des signaux de données de sortie ;
dans laquelle un pilote (16) couplé à un générateur de signaux acoustiques (14) d'une unité périphérique (12) est adapté pour réaliser une procédure d'initialisation comprenant l'établissement d'une communication avec un dispositif externe (42) par le biais d'un support de communication hors corps, l'acquisition à partir du dispositif externe d'une clé sécurisée et le pilotage de la transmission acoustique de la clé sécurisée au dispositif portable au moyen du générateur de signaux acoustiques ; et
**caractérisée en ce que** le récepteur de signal acoustique comprend un réseau bidimensionnel (28) d'éléments de capteur acoustique (30) présentant des parties de capteur exposées au niveau d'une surface de contact avec le corps (32) de l'unité montable sur le corps (20).

14. Unité montable sur le corps (20) selon la revendication 13, dans laquelle l'unité périphérique (12) comprenant le générateur de signaux acoustiques (14) et le pilote couplé (16) est adaptée pour piloter une génération de signaux acoustiques codés en données par le générateur de signaux acoustiques sur la base de signaux de données d'entrée.

15. Unité montable sur le corps (20) selon la revendication 13 ou 14, dans laquelle l'unité montable sur le corps (20) est au moins l'une : d'une unité sur le corps pour un montage sur une partie externe du corps du sujet ou d'une unité dans le corps pour une insertion ou une implantation dans le corps d'un sujet.
